# EUROPÄISCHE PATENTSCHRIFT

(11) **EP 3 044 200 B1**
(45) Veröffentlichungstag und Bekanntmachung des Hinweises auf die Patenterteilung: **09.10.2019**
(21) Anmeldenummer: 14758295.1
(22) Anmeldetag: 29.08.2014
(51) Int. Cl.: C07C 67/08, C07C 67/58, C07C 67/28, C07C 69/33

(54) **VERFAHREN ZUR NACHBEHANDLUNG VON POLYOLESTERN**
METHOD FOR POST-TREATING POLYOL ESTERS
PROCÉDÉ DE POST-TRAITEMENT D'ESTERS DE POLYOLS

(30) Priorität: 14.09.2013 DE 102013015289
(43) Veröffentlichungstag der Anmeldung: 20.07.2016
(73) Patentinhaber: OXEA GmbH, 46147 Oberhausen (DE)
(72) Erfinder: KUBITSCHKE, Jens, 45127 Essen (DE); KREICKMANN, Thorsten, 45239 Essen (DE); ARNOLD, Jörg, 46535 Dinslaken (DE); KRAMER, Matthias, 46244 Bottrop (DE); STRUTZ, Heinz, 47445 Moers (DE)
(86) Internationale Anmeldenummer: PCT/EP2014/002350
(87) Internationale Veröffentlichungsnummer: WO 2015/036090

(56) Entgegenhaltungen:
- WO-A2-2007/095262
- DE-A1- 3 012 203

## Beschreibung

Die Erfindung betrifft ein Verfahren zur Nachbehandlung von Polyolestern, hergestellt durch Umsetzung von linearen oder verzweigten aliphatischen Monocarbonsäuren mit 3 bis 20 Kohlenstoffatomen und Polyolen in Gegenwart einer Lewissäure enthaltend mindestens ein Element der Gruppen 4 bis 14 des Periodensystems der Elemente als Katalysator und in Gegenwart eines Adsorbens, durch anschließende Nachbehandlung des Rohesters durch Zugabe von Wasser bei einer Temperatur unterhalb des Siedepunktes von Wasser bei dem jeweiligen Druck.

Ester mehrwertiger Alkohole, auch Polyolester genannt, finden in großem Umfang und in vielfältiger Weise Anwendung in der Technik, wie zum Beispiel als Weichmacher oder Schmiermittel. Durch die Auswahl geeigneter Ausgangsprodukte lassen sich die physikalischen Stoffeigenschaften, wie zum Beispiel Siedepunkt oder Viskosität, gezielt einstellen und den chemischen Eigenschaften, wie der Hydrolyseresistenz oder der Stabilität gegenüber einem oxidativen Abbau, Rechnung tragen. Auch auf die Lösung konkreter anwendungstechnischer Probleme können Polyolester gezielt zugeschnitten werden. Ausführliche Übersichten über den Einsatz von Polyolestern finden sich zum Beispiel in Ullmann's Encyclopedia of Industrial Chemistry, 5. Auflage, 1985, VCH Verlagsgesellschaft, Vol. A1, Seiten 305-319; 1990, Vol. A15, Seiten 438-440 oder in Kirk Othmer, Encyclopedia of Chemical Technology, 3. Auflage, John Wiley & Sons, 1978, Vol. 1, Seiten 778-787; 1981, Vol. 14, Seiten 496-498.

Die Verwendung von Polyolestern als Schmierstoffe besitzt eine große technische Bedeutung und sie werden besonders für solche Anwendungsgebiete eingesetzt, in denen Schmierstoffe auf Mineralölbasis die gesetzten Anforderungen nur unvollständig erfüllen. Polyolester werden insbesondere als Turbinenmotoren- und Instrumentenöle benutzt. Polyolester für Schmiermittelanwendungen basieren häufig auf 1,3-Propandiol, 1,3-Butandiol, 1,4-Butandiol, 1,2-Hexandiol, 1,6-Hexandiol, Neopentylglykol, Trimethylolpropan, Pentaerythrit, 2,2,4-Trimethylpentan-1,3-diol, Glycerin oder 3(4),8(9)-Dihydroxymethyltricyclo[5.2.1.0^{2.6}]decan, auch als TCD-Alkohol DM bezeichnet, als Alkoholkomponente.

Auch als Weichmacher werden Polyolester in erheblichem Umfang verwendet. Weichmacher finden in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u. a. mit dem Ergebnis, dass seine mechanischen Eigenschaften optimiert, z. B. Formveränderungsvermögen, Elastizität, Festigkeit erhöht werden und die Härte verringert wird.

Eine spezielle Klasse von Polyolestern (sie werden kurz als G-Ester bezeichnet) enthält als Alkoholkomponente Diole bzw. Etherdiole, beispielsweise Ethylenglykol, Diethylenglykol, Triethylenglykol, Tetraethylenglykol, 1,2-Propylenglykol oder höhere Propylenglykole. Ihre Herstellung kann auf unterschiedlichen Wegen erfolgen. Neben der Umsetzung von Alkohol und Säure gegebenenfalls in Gegenwart saurer Katalysatoren werden in der Praxis weitere Prozesse zur Gewinnung von G-Estern angewandt, u. a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuss und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d. h. hohe Ausbeuten erzielt werden.

Umfassende Angaben zur Herstellung von Estern mehrwertiger Alkohole, darunter auch Estern aus Ethylenglykolen und Fettsäuren und zu den Eigenschaften ausgewählter Vertreter dieser Verbindungsklassen finden sich in Goldsmith, Polyhydric Alcohol Esters of Fatty Acids, Chem. Rev. 33, 257 ff. (1943). Beispielsweise erfolgt die Herstellung von Estern des Diethylenglykols, des Triethylenglykols und von Polyethylenglykolen über Reaktionszeiten von 2,5 bis 8 Stunden bei Temperaturen von 130 bis 230°C. Als geeignete Katalysatoren für die Veresterung mehrwertiger Alkohole werden anorganische Säuren, saure Salze, organische Sulfonsäuren, Acetylchlorid, Metalle oder amphotere Metalloxide genannt. Die Entfernung des Reaktionswassers erfolgt mit Hilfe eines Schleppmittels, beispielsweise Toluol oder Xylol oder durch Einleiten inerter Gase wie Kohlendioxid oder Stickstoff. Auf die Gewinnung und die Eigenschaften von Fettsäureestern der Polyethylenglykole geht Johnson (Edit.), Fatty Acids in Industry (1989) Kap. 9, Polyoxyethylene Esters of Fatty Acid ein und gibt eine Reihe präparativer Hinweise. Höhere Diesterkonzentrationen erzielt man durch die Erhöhung des molaren Verhältnisses von Carbonsäure zu Glykol. Geeignete Maßnahmen zur Entfernung des Reaktionswassers sind die Azeotropdestillation in Gegenwart eines mit Wasser nicht mischbaren Lösungsmittels, das Erhitzen unter Durchleiten eines Inertgases oder die Durchführung der Reaktion unter Vakuum in Gegenwart eines Trockenmittels. Verzichtet man auf den Zusatz von Katalysatoren, so sind längere Reaktionszeiten und höhere Reaktionstemperaturen erforderlich. Beide Reaktionsbedingungen können durch den Einsatz von Katalysatoren gemildert werden. Neben Schwefelsäure sind organische Säuren wie p-Toluolsulfonsäure sowie Kationenaustauscher vom Polystyroltyp die bevorzugten Katalysatoren. Auch die Verwendung von Metallpulvern, wie Zinn oder Eisen wird beschrieben. Nach der Lehre von US 2,628,249 lassen sich Farbprobleme bei der Katalyse mit Schwefelsäure oder Sulfonsäure mildern, wenn in Gegenwart von Aktivkohle gearbeitet wird.

Als weitere metallhaltige Katalysatoren werden auch Titan-, Zirconium- oder Zinnalkoholate, -carboxylate oder -chelate zur Herstellung von Polyolestern, beispielsweise gemäß US 5,324,853 A1, eingesetzt. Solche Metallkatalysatoren kann man als Hochtemperaturkatalysatoren ansehen, denn sie erreichen ihre volle Aktivität erst bei hohen Veresterungstemperaturen, im Allgemeinen oberhalb von 180°C. Sie werden häufig nicht zu Beginn der Veresterungsreaktion zugesetzt sondern nachdem das Reaktionsgemisch bereits aufgeheizt wurde und zum Teil unter Wasserabspaltung reagiert hat. Trotz der im Vergleich zur klassischen Schwefelsäurekatalyse erforderlichen höheren Reaktionstemperaturen und längeren Reaktionszeiten werden bei der Katalyse mit solchen metallhaltigen Verbindungen Rohester mit einer vergleichsweise geringen Farbzahl erhalten. Gängige Veresterungskatalysatoren sind beispielsweise Tetra(isopropyl)orthotitanat, Tetra(butyl)orthotitanat, Tetra(butyl)zirconat oder Zinn(II)-2-ethylhexanoat.
Metallspuren in den aufgereinigten Polyolestern können ihre Anwendung als Weichmacher oder Schmiermittel beeinträchtigen, da beispielsweise die elektrische Leitfähigkeit oder die Stabilität gegenüber Luftsauerstoff beeinflusst wird. Der Stand der Technik schlägt eine Reihe von Maßnahmen vor, um den Veresterungskatalysator in gut abtrennbare Folgeprodukte zu überführen.

Nach der in DE 10 2009 048 775 A1 beschriebenen Arbeitsweise wird die Veresterung von Polyolen mit aliphatischen Monocarbonsäuren mit einem Lewissäure-Katalysator in Gegenwart eines Adsorbens durchgeführt. Im Zuge der Aufarbeitung des Rohesters wird mit Wasserdampf behandelt, wobei in ihrem Verlauf noch vorhandener Lewissäure-Katalysator zerstört wird. Durch Filtration zusammen mit dem Adsorbens können die Katalysatorfolgeprodukte auf einfache Weise entfernt werden. Die Wasserdampfbehandlung wird bei Temperaturen im Allgemeinen von 100 bis 250°C und über einen Zeitraum von 0,5 bis 5 Stunden durchgeführt. Während der Aufheizperiode bis zum Erreichen der Arbeitstemperatur hat man möglichst schonend vorzugehen, um eine zu starke thermische Belastung des Rohesters zu vermeiden. Insbesondere bei der Herstellung von Polyolestern auf Basis von Etherdiolen wie zum Beipiel Triethylenglykol oder Tetraethylenglykol sind die Bedingungen der Wasserdampfbehandlung gezielt einzustellen, um einen unerwünschten Abbau der Etherkette zu Nebenprodukten zu unterbinden. Des Weiteren ist die Wasserdampfbehandlung zur Zerstörung des Lewissäure-Katalysators zeitintensiv und beeinträchtigt den pro Reaktorvolumen und Zeit erzielten Produktausstoß.

Es ist ebenfalls bekannt, durch Wasserzugabe und anschließende Behandlung mit alkalischen Reagenzien den Lewissäure-Katalysator in gut abtrennbare Folgeprodukte zu überführen. Nach der in DE 30 12 203 offenbarten Arbeitsweise wird der Rohester mit 5 bis 50 Gew.-% Wasser, bezogen auf die Rohestermenge, versetzt und anschließend erhitzt. Durch die Wärmebehandlung mit Wasser werden gut kristallisierte Folgeprodukte des Lewissäure-Katalysators gebildet. An die Wasserbehandlung schließt sich dann die Behandlung mit Alkali an.

WO 2007/095262 A2 behandelt die Veresterung von 1,3-Propandiol, das aus nachwachsenden Rohstoffen gewonnen wird, mit Fettsäuren, die 8 bis 40 Kohlenstoffatome im Molekül enthalten, in Gegenwart von metallhaltigen Katalysatoren. Nach beendeter Umsetzung kann der Katalysator durch Behandlung mit Wasser entfernt werden.

Aus DE 10 2009 060 865 A1 ist ein Verfahren zur Herstellung von Polyolestern bekannt, das in Gegenwart von Zinnverbindungen durchgeführt wird. Der erhaltene Rohester wird durch Wasserzugabe nachbehandelt. Nach Abtrennen der wässrigen Phase wird der Polyolester gegebenenfalls mit einem Sorptionsmittel behandelt.

DE 40 02 949 A1 offenbart ein Verfahren zur Aufarbeitung eines rohen Veresterungsgemisches, das durch kontinuierliche Veresterung in Gegenwart von metallhaltigen Katalysatoren erhalten wird. Nach Abdestillieren des nicht umgesetzten Alkohols wird der Ansatz gekühlt, mit Aktivkohle versetzt und der Restalkohol in Gegenwart der Aktivkohle mit Wasserdampf oder Stickstoff abgestreift.

Gemäß der Arbeitsweise aus US 5,324,853 A1 versetzt man das rohe Veresterungsgemisch mit einer wässrigen Sodalösung und gegebenenfalls mit Aktivkohle. Durch diese Arbeitsweise werden die Metallverbindungen zu unlöslichen Feststoffen hydrolysiert und können vor der weiteren Aufarbeitung der rohen Esterverbindung abfiltriert werden.

Auch bei der Aufarbeitung eines Rohestergemisches, das durch Umsetzung von mehrbasischen Carbonsäuren mit Monoalkoholen in Gegenwart von Lewissäure-Katalysatoren, beispielsweise von titan- oder zinnenthaltenden Katalysatoren erhalten wird, empfiehlt der Stand der Technik eine Wasserbehandlung zur Katalysatorentfernung. Nach US 5, 434,294 wird der Rohester mit einer wässrigen, alkalischen Lösung bei Temperaturen zwischen 80 bis 150°C behandelt und anschließend über ein Adsorbens filtriert. Bei dem in DE 1 945 359 beschriebenen Aufarbeitungsverfahren wird der Rohester zunächst mit Alkali behandelt und der freie Alkohol durch eine Wasserdampfdestillation entfernt. Anschließend wird das Produkt auf eine Temperatur unterhalb des Siedepunktes des Wassers abgekühlt und anschließend mit mindestens 0,5 Gew.-% Wasser, bezogen auf das aufzuarbeitende Produkt, versetzt. Mittels dieser Wasserbehandlung werden gut filtrierbare Niederschläge der Katalysatorfolgeprodukte erhalten.

Um nach beendeter Veresterungsreaktion eine ausreichende Abtrennung des Lewissäure-Katalysators in Form von Katalysatorfolgeprodukten sicherzustellen, lehrt der Stand der Technik eine Wasserdampfbehandlung in Gegenwart eines Adsorbens oder eine Behandlung mit Wasser bei Temperaturen unterhalb des Siedepunktes von Wasser bei dem jeweiligen Druck in Verbindung mit einer Behandlung basisch reagierender Verbindungen. Da jedoch die Wasserdampfbehandlung zeitaufwendig ist und die Bedingungen gezielt einzustellen sind sowie durch eine Behandlung mit Basen, beispielsweise mit Natriumhydroxid oder Natriumcarbonat, zusätzlich Salze eingetragen werden, die im Verlauf der Rohesteraufarbeitung wieder entfernt werden müssen, besteht daher Bedarf an einem Verfahren zur Nachbehandlung von Polyolestern, das weniger zeitintensiv ist aber gleichzeitig den gewünschten Polyolester in ausreichender Qualität bereitstellt, so dass die geforderten Spezifikationswerte wie Restsäurezahl, Wassergehalt, Hydroxylzahl und Metallrestgehalt sicher eingehalten werden und die Polyolester möglichst vielseitig angewendet werden können.

Die Erfindung besteht daher in einem absatzweisen Verfahren zur Nachbehandlung von Polyolestern, hergestellt durch Umsetzung von Polyolen der allgemeinen Formel (II)

H-(-O-[-CR¹R²-]ₘ-)ₒ-OH (II)

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit überschüssigen linearen oder verzweigten aliphatischen Monocarbonsäuren mit 3 bis 20 Kohlenstoffatomen, die einen geringeren Siedepunkt aufweisen als die eingesetzten Polyole, in Gegenwart einer Lewissäure ausgewählt aus der Gruppe von Titan, Zirkonium, Hafnium, Eisen, Zink, Bor, Aluminium oder Zinn als Elemente oder in Form ihrer Verbindungen als Katalysator und in Gegenwart eines Adsorbens für schwerlösliche Katalysator-Umwandlungsprodukte, in einer Menge von auf 100 Gewichtsteilen Reaktionsansatz 0,1 bis 5, vorzugsweise 0,5 bis 1,5 Gew.-Teile unter Entfernung des gebildeten Wassers, dadurch gekennzeichnet, dass man die überschüssige Monocarbonsäure destillativ abtrennt und dem erhaltenen Rohester Wasser bei einer Temperatur unterhalb des Siedepunktes von Wasser bei dem jeweiligen Druck zusetzt und den mit Wasser versetzten Rohester unter Vermeidung von basisch reagierenden Verbindungen nachbehandelt und von den schwerlöslichen Umwandlungsprodukten und dem in der Veresterungsreaktion anwesenden Adsorbens filtriert.

Die Reaktion zwischen den Ausgangsverbindungen Polyol und der aliphatischen Monocarbonsäure setzt in Abhängigkeit von den Einsatzmaterialien im Bereich von etwa 120 bis 180°C ein und kann anschließend auf unterschiedlich ausgestaltete Weise zu Ende geführt werden.

Nach einer Ausgestaltung der Veresterungsreaktion wird zunächst ausgehend von Raumtemperatur auf eine Temperatur bis auf maximal 280°C, vorzugsweise bis auf 230°C erhitzt und bei konstant gehaltener Temperatur der Druck ausgehend von Normaldruck stufenweise erniedrigt, um die Entfernung des Reaktionswassers zu erleichtern. Die Wahl der Druckstufen, ob ein-, zwei- oder mehrstufig, sowie der auf der jeweiligen Stufe einzustellende Druck kann über einen weiten Bereich variiert und den jeweiligen Bedingungen angepasst werden. Beispielsweise kann in einer ersten Stufe der Druck ausgehend von Normaldruck zunächst bis auf 600 hPa erniedrigt werden und anschließend die Reaktion bei einem Druck von 300 hPa zu Ende geführt werden. Bei diesen Druckangaben handelt es sich um Richtwerte, die zweckmäßig eingehalten werden.

Neben der Variation des Drucks kann ebenfalls auch die Temperatur ausgehend von Raumtemperatur während der Veresterungsreaktion ein-, zwei- oder mehrstufig verändert werden, so dass bei konstant eingestelltem Druck die Temperatur von Stufe zu Stufe erhöht wird, üblicherweise bis auf eine maximale Temperatur von 280°C. Es hat sich aber als zweckmäßig erwiesen, bei von Stufe zu Stufe ansteigender Temperatur auf maximal 280°C zu erhitzen und auch den Druck von Stufe zu Stufe zu erniedrigen. Beispielsweise kann die Veresterungsreaktion ausgehend von Raumtemperatur in einer ersten Stufe bei einer Temperatur bis auf 190°C geführt werden. Ebenfalls wird ein verminderter Druck bis auf 600 hPa angelegt, um das Austreiben des Reaktionswassers zu beschleunigen. Nach Erreichen der Temperaturstufe von 190°C wird der Druck nochmals bis auf 300 hPa erniedrigt und die Veresterungsreaktion bei einer Temperatur bis auf 230°C zu Ende geführt. Bei diesen Temperatur- und Druckangaben handelt es sich um Richtwerte, die zweckmäßiger Weise eingehalten werden. Die einzustellenden Temperatur- und Druckbedingungen auf den jeweiligen Stufen, die Zahl der Stufen sowie die jeweilige Temperaturerhöhungs- oder Druckminderungsrate pro Zeiteinheit können über einen weiten Bereich variiert werden und entsprechend den physikalischen Eigenschaften der Ausgangsverbindungen und der Reaktionsprodukte angepasst werden, wobei die Temperatur- und Druckbedingungen der ersten Stufe ausgehend von Normaldruck und Raumtemperatur eingestellt werden. Besonders zweckmäßig hat es sich erwiesen, die Temperatur in zwei Stufen zu erhöhen und den Druck in zwei Stufen zu erniedrigen.

Die untere Grenze des einzustellenden Drucks hängt von den physikalischen Eigenschaften, wie Siedepunkte und Dampfdrücke, der Ausgangsverbindungen sowie der gebildeten Reaktionsprodukte ab und wird auch durch die Anlagenausstattung festgelegt. Ausgehend von Normaldruck kann innerhalb dieser Grenzwerte stufenweise mit von Stufe zu Stufe abnehmenden Drücken gearbeitet werden. Die obere Temperaturgrenze, üblicherweise 280°C, ist einzuhalten, um die Bildung von Zersetzungsprodukten, die u. a. farbschädigend wirken, zu vermeiden. Die untere Grenze der Temperaturstufen wird durch die Reaktionsgeschwindigkeit bestimmt, die noch ausreichend hoch sein muss, um die Veresterungsreaktion innerhalb einer vertretbaren Zeit abzuschließen. Innerhalb dieser Grenzwerte kann stufenweise mit von Stufe zu Stufe ansteigenden Temperaturen gearbeitet werden.

Die jeweiligen Reaktionsbedingungen, wie Temperatur, Reaktionszeit, anzulegender Druck oder einzusetzender Katalysator sind auf den jeweiligen Polyolester zuzuschneiden, um bei einer ausreichenden Reaktionsgeschwindigkeit die Bildung farbgebender Komponenten in den Hintergrund zu drängen und Abbaureaktionen des Polyolesters möglichst zu vermeiden. Insbesondere bei Polyolestern auf Basis von Etherdiolen, wie zum Beispiel Triethylenglykol oder Tetraethylenglykol, kann ein verstärkter Abbau des Ethergerüstes einsetzen, wenn die Reaktionsbedingungen, wie Temperatur, Reaktionszeit und Katalysatorart und -menge, nicht gezielt auf den jeweiligen Polyolester eingestellt werden.

Bei der Veresterung lässt man das Polyol mit überschüssiger Monocarbonsäure reagieren, die einen geringeren Siedepunkt aufweist als das eingesetzte Polyol und die bei der nachfolgenden Aufarbeitung des Rohesters auf einfache Weise destillativ abgetrennt werden kann. Die aliphatische Monocarbonsäure wird in einem 10 bis 50%-igen molaren, vorzugsweise in einem 20
bis 40%-igen molaren Überschuss je Mol zu veresternder Hydroxylgruppe des Polyols eingesetzt.

Das gebildete Reaktionswasser wird im Laufe der Veresterungsreaktion zusammen mit der überschüssigen Monocarbonsäure aus dem Reaktionsgefäß abdestilliert und in einen nachgeschalteten Phasentrenner geleitet, in dem sich Monocarbonsäure und Wasser je nach ihren Löslichkeitseigenschaften auftrennen. Zwischen Reaktionsgefäß und Phasentrenner kann ebenfalls eine Fraktionskolonne mit 1 bis 25, vorzugsweise 2 bis 10 und insbesondere 3 bis 6 theoretischen Böden installiert werden, in der die wasserangereicherte Fraktion über den Kolonnenkopf in den Phasentrenner geleitet wird und die monocarbonsäureangereicherte Fraktion über den Kolonnenboden in das Reaktionsgefäß zurückfließt.

Gegebenenfalls bildet die eingesetzte Monocarbonsäure mit Wasser unter den Reaktionsbedingungen auch ein Azeotrop und vermag als Schleppmittel das Reaktionswasser zu entfernen. Aus dem Wasseranfall kann der Reaktionsverlauf verfolgt werden. Das abgeschiedene Wasser wird aus dem Prozess entfernt, während die Monocarbonsäure aus dem Phasentrenner wieder in das Reaktionsgefäß zurückfließt. Die Zugabe eines weiteren organischen Lösungsmittels, wie Hexan, 1-Hexen, Cyclohexan, Toluol, Xylol oder Xylolisomerengemische, das die Aufgabe des Azeotropbildners übernimmt, ist nicht ausgeschlossen, jedoch auf wenige Ausnahmefälle beschränkt. Der Azeotropbildner kann bereits zu Beginn der Veresterungsreaktion oder nach dem Erreichen höherer Temperaturen zugesetzt werden. Wenn die theoretisch zu erwartende Wassermenge angefallen ist oder die Hydroxylzahl, beispielsweise bestimmt nach DIN 53240, unter einen festgelegten Wert gefallen ist, beendet man die Reaktion und beginnt mit der Aufarbeitung des Rohesters.

Als Katalysatoren für die Veresterung des Polyols mit der Monocarbonsäure verwendet man Lewissäuren ausgwählt aus der Gruppe Titan, Zirkonium, Hafnium, Eisen, Zink, Bor, Aluminium oder Zinn, die als Element in fein verteilter Form oder bevorzugt in Form von Verbindungen verwendet werden und die in fester oder flüssiger Form eingesetzt werden können. Unter dem Begriff Lewissäure im Sinne der Erfindung wird die allgemein übliche Definition für solche Elemente oder Verbindungen verstanden, die eine Elektronenlücke aufweisen, wie beispielsweise in Römpp's Chemie-Lexikon, 8. Auflage, Franck'sche Verlagshandlung 1983, Band 3, H-L ausgeführt. Geeignete Verbindungen sind beispielsweise Zinn(II)-oxid, Zinn(IV)oxid, Zinn-Carboxylate, wie Zinn(II)-2-ethylhexanoat, Zinn(ll)-oxalat, Zinn(II)-acetat oder Zinn(IV)-acetat, Zinn(IV)-alkoholate, wie Tetra(methyl)stannat, Tetra(ethyl)stannat, Tetra(propyl)stannat, Tetra(iso-propyl)stannat oder Tetra(isobutyl)stannat, oder Organozinnverbindungen, wie Butylzinnmaleat oder Dibutylzinndilaurat.

Zu den geeigneten Titanverbindungen zählen Alkoholate, wie Tetra(methyl)orthotitanat, Tetra(ethyl)orthotitanat, Tetra(propyl)orthotitanat, Tetra(iso-propyl)orthotitanat, Tetra(butyl)orthotitanat, Tetra(iso-butyl)orthotitanat, Tetra(pentyl)orthotitanat oder Tetra(2-ethylhexyl)orthotitanat; Acylate, wie Hydroxytitanacetat, Hydroxytitanbutyrat oder Hydroxytitanpentanoat; Carboxylate wie Titan(IV)-acetat, Titan(IV)-propionat, Titan(IV)-butyrat, Titan(IV)-pentanoat oder Titan(IV)-2-ethylhexanoat; oder Chelate, wie Tetraethylenglykoltitanat oder Tetrapropylenglykoltitanat. Auch die entsprechenden Zirkonium- oder Hafniumverbindungen können mit Erfolg eingesetzt werden, wie Tetra(methyl)orthozirkonat, Tetra(ethyl)orthozirkonat, Tetra(propyl)orthozirkonat, Tetra(iso-propyl)orthozirkonat, Tetra(butyl)orthozirkonat, Tetra(iso-butyl)orthozirkonat, Tetra(pentyl)orthozirkonat oder Tetra(2-ethylhexyl)orthozirkonat.

Geeignet sind ebenfalls Borsäure sowie Borsäureester, wie Borsäuretrimethylester, Borsäuretriethylester, Borsäuretripropylester, Borsäuretriisopropylester, Borsäuretributylester oder Borsäuretriisobutylester.

Ebenfalls geeignet sind Aluminiumoxid, Aluminiumhydroxid, Aluminiumcarboxylate, wie Aluminiumacetat oder Aluminiumstearat, oder Aluminiumalkoholate wie Aluminiumtributylat, Aluminium-tri-sec.-butylat, Aluminium-tri-tert.-butylat oder Aluminium-tri-isopropylat.

Auch Zinkoxid, Zinksulfat und Zinkcarboxylate wie Zinkacetat Dihydrat oder Zinkstearat, und Eisen(II)acetat oder Eisen(III)hydroxid-oxid können als Katalysatoren eingesetzt werden.

Der Katalysator kann dem Reaktionsgemisch bereits zu Beginn zugesetzt werden oder erst nachträglich unter Beachtung von Sicherheitsmaßnahmen bei erhöhter Temperatur, wenn beispielsweise die Abtrennung des Reaktionswassers eingesetzt hat. Der Katalysator kann dabei in einer Portion oder portionsweise zugesetzt werden. Besonders empfehlenswert ist, gegen Ende der Veresterungsreaktion noch eine Restmenge an Katalysator zuzusetzen.

Die Menge des zugesetzten Veresterungskatalysators beträgt 1X10⁻⁵ bis 20 mol-%, vorzugsweise 0,01 bis 5 mol-%, insbesondere 0,01 bis 2 mol-%, bezogen auf die im Unterschuss zugesetzte Ausgangsverbindung, zweckmäßigerweise bezogen auf Polyol. Bei höheren Katalysatormengen ist mit Spaltungsreaktionen der Polyolester zu rechnen.

Besonders bei der Herstellung von Polyolestern auf Basis von Etherdiolen, wie zum Beispiel Triethylenglykol oder Tetraethylenglykol, ist bei dem Einsatz hoher Katalysatorkonzentrationen gegen Reaktionsende und in der Phase des Umsatzes letzter Reste an freien Hydroxylgruppen eine verstärkte Spaltung der Etherkette zu befürchten, so dass in diesem Falle die Reaktionstemperatur oder der anzulegende Druck anzupassen sind. Je höher die gewählte Katalysatorkonzentration ist, desto niedriger ist im Allgemeinen die Reaktionstemperatur oder der anzulegende Druck zu wählen und es ist nach einem optimierten Temperatur- und Druckprofil zu arbeiten. Bei zu niedrigen Katalysatorkonzentrationen wird die Veresterungsgeschwindigkeit so gering, dass kein akzeptabler Umsatz in einer vertretbaren Reaktionszeit beobachtet wird.

Die Zugabe des Veresterungskatalysators kann in flüssiger oder fester Form erfolgen. Feste Katalysatoren, beispielsweise Zinn(II)oxid, Zinkoxid oder Eisen(III)hydroxid-oxid werden nach beendeter Veresterungsreaktion im Zuge der weiteren Aufarbeitung abgetrennt. Werden die Veresterungskatalysatoren als flüssige Verbindungen zugesetzt, beispielsweise Tetra(iso-propyl)orthotitanat oder Tetra(butyl)orthotitanat, die nach beendeter Veresterungsreaktion im Reaktionsgemisch noch gelöst vorliegen, so werden diese Verbindungen durch die erfindungsgemäße Nachbehandlung in schwer lösliche Umwandlungsprodukte überführt, die zusammen mit dem in der Veresterungsreaktion anwesenden Adsorbens durch Filtration auf einfache Weise abgetrennt werden können.

Die Veresterung wird in Gegenwart eines Adsorbens durchgeführt. Man verwendet dabei poröse, großflächige feste Materialien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselguhr, oberflächenreiche Aluminiumoxide und Aluminiumoxidhydrate, mineralische Materialien, wie Tone oder Carbonate, oder Aktivkohle. Besonders bewährt hat sich Aktivkohle. Im Allgemeinen ist das Adsorbens feinteilig in der Reaktionslösung suspendiert, die durch intensives Rühren oder durch Einleiten eines Inertgases bewegt wird. Dadurch wird ein inniger Kontakt zwischen der flüssigen Phase und dem Adsorbens erreicht. Die Menge des Adsorbens kann weitgehend frei und somit den individuellen Erfordernissen entsprechend eingestellt werden. Bezogen auf 100 Gewichtsteile des flüssigen Reaktionsansatzes setzt man 0,1 bis 5, vorzugsweise 0,5 bis 1,5 Gewichtsteile des Adsorbens ein.

Wegen der eingangs beschriebenen Qualitätskriterien für Polyolester sind die Verfahrensschritte bei der Veresterungsstufe unter Entfernung des Reaktionswassers und bei der Aufarbeitung des Rohesters sehr wesentliche Prozessmerkmale, denn die Abstimmung dieser Verfahrensschritte beeinflusst in wesentlichem Maße die sensorischen und optischen Eigenschaften der Endprodukte sowie den Restgehalt an Katalysator. Insbesondere werden durch eine optimierte Prozessführung Polyolester auf Basis von Etherdiolen, beispielsweise Triethylenglykol oder Tetraethylenglykol, mit hoher Reinheit sowie geringer Farbzahl und hoher Farbstabilität gewonnen. Die Struktur der Ausgangsstoffe, der mehrwertigen Alkohole und der aliphatischen Monocarbonsäuren, ist dagegen für die mechanischen und thermischen Eigenschaften der mit den Polyolestern weichgestellten Kunststoffmassen maßgebend und beeinflusst die Hydrolyse- und Oxidationsstabilität von Schmiermitteln.

Das nach Beendigung der Umsetzung anfallende Reaktionsgemisch enthält neben dem Polyolester als erwünschtem Reaktionsprodukt nicht umgesetzte Ausgangsstoffe, insbesondere noch überschüssige aliphatische Monocarbonsäure, da bei der Veresterungsreaktion mit einem Monocarbonsäureüberschuss gearbeitet wird. Üblicherweise destilliert man zunächst unumgesetzte und im Überschuss vorliegende Ausgangsverbindungen ab, zweckmäßigerweise unter Anlegen eines verminderten Drucks.

Anschließend wird dem Rohester unterhalb des Siedepunktes von Wasser bei dem jeweiligen Druck Wasser zugesetzt. Die zugesetzte Wassermenge ist vergleichsweise gering und liegt bei 0,5 bis 5 Gew.-%, vorzugsweise 1 bis 4 Gew.-%, bezogen auf den Reaktionsansatz. Dabei empfiehlt es sich, nach Abtrennung der überschüssigen Ausgangsverbindung und nach destillativer Abtrennung der überschüssigen Monocarbonsäure, den Reaktionsansatz bei Normaldruck auf eine Temperatur im Bereich von 40 bis unterhalb100°C, vorzugsweise auf 60 bis 90°C abkühlen zu lassen und Wasser zuzugeben. Da die zugesetzte Wassermenge, bezogen auf den Reaktionsansatz, vergleichsweise gering ist, wird das zugesetzte Wasser nur in Ausnahmefällen vorgeheizt. Die anschließende Wasserbehandlung erfolgt in dem Temperaturbereich von 40 bis unterhalb 100°C, vorzugsweise 60 bis 90°C über einen Zeitraum von 10 Minuten bis 4 Stunden, vorzugsweise 30 Minuten bis 2 Stunden durch intensives Rühren, Umwälzen oder durch Einleiten eines Inertgases. Bei der Wasserbehandlung nutzt man die bereits in den Rohester eingebrachte Wärmeenergie, beispielsweise die während der Veresterungsreaktion eingetragene Aufheizenergie oder die während der destillativen Abtrennung der überschüssigen Ausgangsverbindung zugeführte Wärmeenergie. Gelegentlich wird auch während der Wasserbehandlung noch zusätzlich Wärmeenergie zugeführt, um den Temperaturbereich von 40 bis unterhalb 100°C, vorzugsweise von 60 bis 90°C, während der Wasserbehandlung aufrechtzuerhalten.

Die Zugabe von Wasser kann ebenfalls bei erhöhtem Druck oberhalb von Normaldruck erfolgen. Die zugesetzte Wassermenge und die Behandlungsdauer entsprechen der Normaldruckfahrweise. Die Behandlungstemperatur liegt bei mindestens 100°C und wird durch den angelegten Druck bestimmt. Die obere Temperaturgrenze kann bis unterhalb des Siedepunktes von Wasser bei dem angelegten Druck eingestellt werden, vorzugsweise von 100 bis 150°C.

Es wurde überraschenderweise festgestellt, dass durch die Nachbehandlung mit Wasser nach der erfindungsgemäßen Arbeitsweise bei vergleichsweise niedrigen Temperaturen der Lewissäure-Katalysator bereits nach einer vergleichsweisen kurzen Behandlungsdauer von 10 Minuten bis 4 Stunden, vorzugsweise 30 Minuten bis 2 Stunden, nahezu vollständig in schwer lösliche Umwandlungsprodukte überführt wird, die leicht abgetrennt werden können.

Gegenüber dem aus DE 10 2009 048 775 A1 bekannten Verfahren, bei dem der Lewissäure-Katalysator mittels einer Wasserdampfbehandlung in Folgeprodukte überführt wird, kann erfindungsgemäß bei der absatzweisen Verfahrensführung die Verweilzeit des Rohesters in der Produktionsanlage deutlich reduziert und damit die Raum-Zeit-Ausbeute an dem gewünschten Polyolester deutlich erhöht werden. Auch wird die Wasserdampfbehandlung bei höheren Temperaturen, vorzugsweise bei Temperaturen von 150 bis 220°C, durchgeführt, so dass es insbesondere bei längeren Behandlungszeiten zu unerwünschten Abbaureaktionen und zu einer Verschlechterung der Farbzahl kommen kann. Insbesondere bei der Herstellung von Polyolestern auf Basis von Etherdiolen, wie zum Beispiel von Triethylenglykol und Tetraethylenglykol, ist bei einer übermäßigen thermischen Belastung mit einem unerwünschten Abbau der Etherkette zu rechnen. Darüber hinaus kann es ebenfalls während der Wasserdampfbehandlung zu einer Spaltung der Estergruppe unter Freisetzung sauer reagierender Verbindungen kommen, so dass eine nachfolgende Behandlung mit basisch reagierenden Substanzen notwendig wird, um den Spezifikationswert für die Neutralisationszahl oder Säurezahl, beispielsweise bestimmt nach DIN EN ISO 3682/ASTM D 1613, sicher einzuhalten. So wird in DE 10 2009 048 775 A1 ausdrücklich darauf hingewiesen, während der Aufheizperiode bis zum Erreichen der Arbeitstemperatur möglichst schonend vorzugehen, um eine übermäßige thermische Belastung des Polyolesters während der Wasserdampfbehandlung zu vermeiden und damit Abbaureaktionen und Farbzahlverschlechterungen zurückzudrängen.

Diese Nachteile lassen sich durch die erfindungsgemäße Behandlung mit Wasser bei einer Temperatur unterhalb des Siedepunktes von Wasser bei dem jeweiligen Druck umgehen. Aufgrund der kürzeren Behandlungsdauer und der niedrigeren Behandlungstemperaturen ist die Gefahr der Farbzahlverschlechterung und der Spaltung der Estergruppe und der Ethergruppe im Falle der Veresterung von Etherdiolen geringer, so dass auf eine Behandlung mit basisch reagierenden Reagenzien im Zuge des weiteren Aufarbeitungsverfahrens des Rohesters verzichtet werden kann. Dadurch vermeidet man vorteilhafterweise den Eintrag von Alkaliionen, beispielsweise von Natriumhydroxid, die anschließend in Form von Salzen abgetrennt werden müssen. Restgehalte an Alkaliionen beeinflussen nachteilig das Isolationsverhalten der gewünschten Polyolester. Bei dem erfindungsgemäßen Nachbehandlungsverfahren wird somit auf den Einsatz von basisch reagierenden Verbindungen zur Erniedrigung der Säure- oder Neutralisationszahl verzichtet.

In einer weiteren Ausgestaltung der erfindungsgemäßen Nachbehandlung wird das Wasser verwendet, das durch Kondensation von zur Wärme- oder Vakuumerzeugung eingesetztem Wasserdampf zurückgewonnen wird. Man bezeichnet häufig dieses beim Betrieb der Veresterungsanlage verwendete und zurückgewonnene Wasser auch als Kondensat. Bei dieser Verfahrensvariante vermeidet man den Einsatz von Frischwasser und somit auch den Anfall zusätzlichen Abwassers. Auch fällt das Kondensat bei einer erhöhten Temperatur an und kann dem Rohester bei der erforderlichen Behandlungstemperatur ohne vorhergehendes zusätzliches Aufheizen zugemischt werden.

Durch die erfindungsgemäße Nachbehandlung mit Wasser wird der Lewissäure-Katalysator hydrolysiert und in unlösliche Katalysatorfolgeprodukte überführt. Der Restgehalt an im Rohester gelösten Fremdstoffen, die durch die Verwendung der Lewissäure-Katalysatoren eingetragen werden, beispielsweise der Titangehalt, wird durch analytische Bestimmungen gemäß ASTM D 5185 zeitlich verfolgt. Sobald der Restgehalt an diesen Fremdstoffen unterhalb eines festgelegten Grenzwertes gefallen ist, wird der Rohester weiter aufgearbeitet.

In einer Verfahrensvariante wird der Rohester zunächst von den unlöslichen Katalysator-Folgeprodukten und dem Adsorbens filtriert und anschließend getrocknet.

Die Filtration erfolgt in konventionellen Filtrierapparaturen bei normaler Temperatur oder bei Temperaturen bis 120°C. Die Filtration kann durch gängige Filtrierhilfsmittel wie Cellulose, Kieselgel, Kieselgur oder Holzmehl unterstützt werden. Ihr Einsatz ist jedoch auf Ausnahmefälle beschränkt.

Es folgt die Trocknung des Polyolesters, beispielsweise in dem man ein inertes Gas durch das Produkt bei erhöhter Temperatur leitet. Auch Wasserdampf kann zusätzlich eingeleitet werden, um den Trocknungsvorgang zu unterstützen. Es kann auch bei erhöhter Temperatur gleichzeitig ein Unterdruck angelegt und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen Trocknungsbedingungen, wie Temperatur, Druck und Dauer lassen sich durch einfache Vorversuche ermitteln. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa und insbesondere 1 bis 20 hPa. Durch die Trocknung, gegebenenfalls zusammen mit dem Einleiten von Wasserdampf, werden Reste an Ausgangsverbindungen, beispielsweise Monocarbonsäure, und Wasser entfernt.

In einer alternativen Verfahrensvariante kann der Rohester in Gegenwart der unlöslichen Katalysatorprodukte und des Adsorbens zunächst getrocknet und dann filtriert werden.

In einigen Fällen kann es sich als vorteilhaft erweisen, nach der Wasserbehandlung und vorzugsweise nach Filtration und vor der Trocknung den erhaltenen Polyolester einer Behandlung mit Wasserstoffperoxid zu unterziehen, beispielsweise gemäß dem in DE 10 2010 027 458 A1 beschriebenen Verfahren mit einer wässrigen Wasserstoffperoxidlösung. In diesem Falle schließt sich dann eine Wasserdampfbehandlung gegebenenfalls mit Trocknung und Feinfiltration an. Die Wasserdampfbehandlung dient zur Zerstörung peroxidischer Verbindungen und zur Entfernung eingetragenen Wassers und wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung erfolgt im Allgemeinen bei Temperaturen von 100 bis 250°C, vorzugsweise von 150 bis 220°C und insbesondere von 170 bis 200°C. Sie wird üblicherweise über einen Zeitraum von 0,5 bis 5 Stunden durchgeführt.

Es werden hellfarbige Polyolester erhalten, die auch den übrigen Spezifikationen, wie Wassergehalt, Restsäuregehalt, Restgehalt an Katalysatorbestandteilen und Restgehalt an Monoester genügen.

Die als Ausgangsstoffe für die Veresterungsreaktion eingesetzten mehrwertigen Alkohole oder Polyole genügen der allgemeinen Formel (II)

H-(-O-[-CR¹R²-]ₘ-)ₒ-OH (II)

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

Als Polyole, die in der Veresterungsreaktion zu hellfarbigen Polyolestern umgesetzt werden können, eignen sich beispielsweise Di-Trimethylolpropan, oder Di-Pentaerythrit.

Als weitere Polyole kommen die Oligomere von Ethylenglykol und 1,2-Propylenglykol, insbesondere die Etherdiole Di-, Tri- und Tetraethylenglykol oder Dipropylenglykol, Tripropylenglykol oder Tetrapropylenglykol in Betracht. Ethylen- und Propylenglykole sind industriell produzierte Chemikalien.

Basissubstanz zu ihrer Herstellung ist Ethylenoxid und Propylenoxid, aus dem man 1,2-Ethylenglykol und 1,2-Propylenglykol durch Erhitzen mit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden. Dipropylenglykol, Tripropylenglykol, Tetrapropylenglykol und höhere Propoxylierungsprodukte sind aus der mehrfachen Addition von Propylenoxid an 1,2-Propylenglykol zugänglich.

Zur Gewinnung von hellfarbigen Polyolestern setzt man in der Veresterungsreaktion lineare oder verzweigte, aliphatische Monocarbonsäuren mit 3 bis 20 Kohlenstoffatomen im Molekül ein. Obgleich man gesättigten Säuren in vielen Fällen den Vorzug gibt, können, in Abhängigkeit vom jeweiligen Anwendungsgebiet der Weichmacher oder Schmiermittel, auch ungesättigte Carbonsäuren als Reaktionskomponente zur Estersynthese verwendet werden. Beispiele für Monocarbonsäuren als Bausteine von Polyolestern sind Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, Cyclohexancarbonsäure, 2-Ethylhexansäure, n-Nonansäure, 2-Methyloctansäure, Isononansäure, 3,5,5-Trimethylhexansäure, 2-Propylheptansäure, 2-Methylundecansäure, Isoundecancarbonsäure, Tricyclodecancarbonsäure und Isotridecancarbonsäure. Besonders bewährt hat sich das neue Verfahren für die Nachbehandlung von Polyolestern der oligomeren Ethylenglykole sowie der oligomeren Propylenglykole mit C₄- bis C₁₃- bzw. C₅- bis C₁₀-Monocarbonsäuren sowie zur Nachbehandlung von Polyolestern auf Basis von Di-Trimethylolpropan.

Die Polyolester des Ethylenglykols sowie seine Oligomeren eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden. Nach dem erfindungsgemäßen Nachbehandlungsverfahren lassen sich auf einfache Weise Polyolester mit ausgezeichneten Farbeigenschaften herstellen, die auch weiteren Qualitätsansprüchen, wie geringem Geruch, einer geringen Säurezahl und geringen Katalysatorverunreinigungen genügen. Besonders eignet sich das erfindungsgemäße Verfahren zur Nachbehandlung von Triethylenglykol-di-2-ethylhexanoat (3G8 Ester), Tetraethylenglykol-di-n-heptanoat (4G7 Ester), Triethylenglykol-di-2-ethylbutyrat (3G6 Ester), Triethylenglykol-di-n-heptanoat (3G7 Ester) oder Tetraethylenglykol-di-2-ethylhexanoat (4G8 Ester).

Das erfindungsgemäße Verfahren wird absatzweise in den für die chemische Technik typischen Reaktionsapparaten durchgeführt. Bewährt haben sich Rührkessel, auch als Rührkesselkaskade, oder Reaktionsrohre.

In den folgenden Beispielen wird das erfindungsgemäße Verfahren näher erläutert.

### Ausführungsbeispiele:

### Beispiel 1:

### Herstellung von Triethylenglykol-di-2-ethylhexanoat (3G8 Ester) mit anschließender Wasserbehandlung

In einem beheizbaren Vierhalskolben, versehen mit Rührer, Innenthermometer und Wasserabscheider, wurden 1,66 mol Triethylenglykol und 4,33 mol 2-Ethylhexansäure vorgelegt. Nach Zugabe von 0,025 Gew.-% des Titan-Katalysators Tyzor® TPT, bezogen auf die Gesamtmasse, und 1 Gew.-% Aktivkohle, bezogen auf Triethylenglykoleinsatz, wurde das Reaktionsgemisch bei 600 hPa auf eine Temperatur von 220°C erhitzt und gebildetes Reaktionswasser abgenommen. Nach einer Reaktionszeit von zwei Stunden bei dieser Stufe wurde der Druck auf 400 hPa erniedrigt und die Temperatur bei 220°C belassen. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Die Gesamtreaktionszeit betrug 7 Stunden. Anschließend wurde die überschüssige 2-Ethylhexansäure destillativ bei einer Sumpftemperatur von 110-210°C und einem Druck von 1 hPa entfernt.

Nach Zugabe von 3 Gew.-% Wasser, bezogen auf den Rohester, wurde das Rohprodukt bei 90°C über 60 Minuten gerührt und anschließend filtriert. Es wurde Triethylenglykol-di-2-ethylhexanoat mit einem Titan-Gehalt unter der Nachweisgrenze von 0,5 ppm erhalten (Bestimmung des Titangehalts gemäß ASTM D 5185). Die Ergebnisse der gaschromatographischen Untersuchung (Gew.-%) sowie die gemessene Neutralisationszahl (DIN EN ISO 3682/ASTM D 1613) sind in der nachfolgenden Tabelle 1 zusammengestellt.

**Tabelle 1: Nachbehandlung von Triethylenglykol-di-2-ethylhexanoat (3G8 Ester) mit Wasser**

| Bezeichnung: | Einsatz | Säureabtrennung | [1-A] |
|---|---|---|---|
| Gaschromatographische Analyse (Gew.-%) | 3G8 Ester nach 7 Stunden Veresterung | 3G8 Ester nach 2-Ethylhexansäureabtrennung | 3G8 Ester nach Wasserbehandlung, 3 Gew.% Wasser, 60 Minuten, 90°C |
| Vorlauf | 0,1 | 0,1 | - |
| 2-Ethylhexansäure | 16,7 | 0,1 | 0,1 |
| Triethylenglykol | 0,1 | - | - |
| Triethylenglykol-mono-2-ethyl-hexanoat | 1,5 | 0,9 | 1,0 |
| Diethylenglykol-di-2-ethylhexanoat | 0,4 | 0,5 | 0,5 |
| Triethylenglykol-di-2-ethylhexanoat | 79,8 | 97,4 | 97,4 |
| Rest | 1,4 | 1,0 | 1,0 |
| | | | |
| Neutralisationszahl (mg KOH/g, DIN EN ISO 3682/ASTM D 1613) | | 0,1 | 0,1 |
| Titangehalt (ppm, ASTM D 5185) | | 20 | <0,5 |

### Beispiel 2:

### Nachbehandlung mit Wasser; Vergleich verschiedener Wassermengen und Behandlungszeiten

Für die nachfolgenden Versuche kam ein gemäß Beispiel 1 hergestelltes Triethylenglykol-di-2-ethylhexanoat zum Einsatz, das nach Abtrennung der überschüssigen 2-Ethylhexansäure einen verhältnismäßig hohen Titan-Gehalt von 80 ppm aufwies. Die für die Nachbehandlung eingesetzten Wassermengen, bezogen auf Rohester, die angewandten Behandlungszeiten und die ermittelten gaschromatographischen Gehalte (Gew.-%) sowie die Hazen Farbzahl nach DIN ISO 6271 und der Titangehalt gemäß ASTM D 5185 sind in der nachfolgenden Tabelle zusammengestellt

**Tabelle 2: Nachbehandlung von Triethylenglykol-di-2-ethylhexanoat (3G8 Ester) mit verschiedenen Wassermengen und bei verschiedenen Behandlungszeiten**

| Bezeichnung: | Einsatz | [2-A] | [2-B] | [2-C] |
|---|---|---|---|---|
| Gaschromatographische Analyse (Gew.-%) | 3G8 Ester | 3G8 Ester | 3G8 Ester | 3G8 Ester |
| | | 10 Gew.-%Wasser | 3 Gew.-% Wasser | 3 Gew.-% Wasser |
| | | 60 Minuten | 30 Minuten | 60 Minuten |
| | | 90°C | 90°C | 90°C |
| Vorlauf | 0,1 | - | 0,1 | 0,1 |
| 2-Ethylhexansäure | 1,1 | 1,1 | 1,2 | 1,2 |
| Triethylenglykol | - | - | - | - |
| Triethylenglykol-mono-2-ethylhexanoat | 0,5 | 0,5 | 0,6 | 0,4 |
| Diethylenglykol-di-2-ethylhexanoat | 0,2 | 0,1 | 0,1 | 0,1 |
| Triethylenglykol-di-2-ethylhexanoat | 97,2 | 97,3 | 97,1 | 97,3 |
| Rest | 0,9 | 1,0 | 0,9 | 0,9 |
| | | | | |
| Farbzahl nach Hazen | 46 | 22 | 29 | 28 |
| (DIN ISO 6271) | | | | |
| Titangehalt | 80 | <0,5 | 1,6 | <0,5 |
| (ppm, ASTM D 5185) | | | | |

Es zeigt sich, dass eine Wassermenge von 3 Gew.-%, bezogen auf die Rohestermenge, bei einer Behandlungsdauer von 1 Stunde bei 90°C ausreichend ist, den hohen Ti-Gehalt auf einen Wert kleiner 0,5 ppm (Nachweisgrenze) zu reduzieren. Die Wasserbehandlung hat weiterhin einen vorteilhaften Einfluss auf die Hazen Farbzahl, die von 46 Einheiten im Einsatzprodukt auf 28 Einheiten (Versuch 2-C) vermindert werden kann.

Anstelle von Frischwasser kann ebenfalls das Wasser, das durch Kondensation von zur Wärme- oder Vakuumerzeugung eingesetztem Wasserdampf zurückgewonnen wird, häufig auch als Kondensat bezeichnet, zur Abreicherung des Titans im Rohester verwendet werden.

In der nachfolgenden Tabelle 3 sind die Bedingungen der Behandlung des Rohesters mit Kondensat sowie die gemäß ASTM D 5185 ermittelten Titangehalte wiedergegeben.

**Tabelle 3: Nachbehandlung von Triethylenglykol-di-2-ethylhexanoat (3G8 Ester) mit Kondensat bei 75°C**

| Bezeichnung: | Einsatz | [2-D] | [2-E] |
|---|---|---|---|
| | | 3 Gew.-% | 3 Gew.-% |
| | | Kondensat | Kondensat |
| | | 45 Minuten | 160 Minuten |
| Titangehalt (ppm, ASTM D 5185) | 9,6 | 0,8 | <0,5 |
| Titangehalt (ppm, ASTM D 5185) | 14,0 | 1,8 | <0,5 |

Wie die Ergebnisse aus der Tabelle 3 zeigen, können auch bei Einsatz von Kondensat die Titanrestgehalte im behandelten Rohester deutlich reduziert werden.

### Beispiel 3:

### Zusätzliche Nachbehandlung mit einer wässrigen Wasserstoffperoxidlösung und unmittelbar anschließender Wasserdampfbehandlung

Gemäß dieser Versuchseinstellung wurde Triethylenglykol-di-2-ethyl-hexanoat (3G8 Ester), das nach Wasserbehandlung einen Titanrestgehalt unterhalb der Nachweisgrenze aufwies, das aber hinsichtlich der Hazen Farbzahl nicht überzeugte, einer Nachbehandlung mit einer wässrigen 30%-igen Wasserstoffperoxidlösung unterzogen. Die applizierte Wasserstoffperoxidmenge betrug 0,1 Gew.-% absolut, bezogen auf den behandelten Rohester. Nach der Wasserstoffperoxidbehandlung erfolgte unmittelbar anschließend eine Wasserdampfbehandlung mit nachfolgender Trocknung. Die angewandten Reaktionsbedingungen, die gaschromatographisch bestimmte Zusammensetzung (Gew.-%) sowie die ermittelten Kennzahlen sind in der nachfolgenden Tabelle 3 zusammengestellt.

**Tabelle 4: Nachbehandlung von Triethylenglykol-di-2-ethylhexanoat (3G8 Ester) mit Wasser und nachfolgende Wasserstoffperoxidbehandlung mit unmittelbar anschließender Wasserdampfbehandlung und Trocknung**

| Bezeichnung: | Einsatz | [3-A] | [3-B] | [3-C] |
|---|---|---|---|---|
| Gaschromatographische Analyse (Gew.-%) | 3G8 Ester | 3G8 Ester | 3G8 Ester | 3G8 Ester |
| | | 10 Gew.-% | 0,1% absolut H₂O₂ | Wasserdampfbehandlung |
| | | Wasser | 60 Minuten | 60 Minuten, 160°C; |
| | | 60 Minuten | 120°C | Trocknung 120 Minuten |
| | | 90°C | | 180°C |
| Vorlauf | 0,1 | 0,1 | 0,1 | - |
| 2-Ethylhexansäure | 1,0 | 0,6 | 0,6 | - |
| Triethylenglykol | - | - | 0,1 | - |
| Triethylenglykol-mono-2-ethylhexanoat | 1,4 | 1,3 | 1,3 | 1,1 |
| Diethylenglykol-di-2-ethylhexanoat | 0,2 | 0,2 | 0,2 | 0,2 |
| Triethylenglykol-di-2-ethylhexanoat | 96,5 | 96,9 | 96,4 | 97,2 |
| Rest | 0,8 | 0,9 | 1,3 | 1,5 |
| | | | | |
| Farbzahl nach Hazen | 86 | 57 | 13 | 17 |
| (DIN ISO 6271) | | | | |
| Neutralisationszahl | 3,1 | 3,1 | 3,2 | 0,1 |
| (mg KOH/g, DIN EN ISO 3682/ASTM D 1613) | | | | |
| Hydroxylzahl | 3,7 | 3,2 | 4,7 | 1,9 |
| (mg KOH/g, DIN 53240) | | | | |
| Titangehalt | 15 | <0,5 | <0,5 | <0,5 |
| (ppm, ASTM D 5185) | | | | |

## Patentansprüche

1. Absatzweises Verfahren zur Nachbehandlung von Polyolestern, hergestellt durch Umsetzung von Polyolen der allgemeinen Formel (II)
H-(-O-[-CR¹R²-]ₘ-)ₒ-OH (II)
in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit überschüssigen linearen oder verzweigten aliphatischen Monocarbonsäuren mit 3 bis 20 Kohlenstoffatomen, die einen geringeren Siedepunkt aufweisen als die eingesetzten Polyole, in Gegenwart einer Lewissäure ausgewählt aus der Gruppe von Titan, Zirkonium, Hafnium, Eisen, Zink, Bor, Aluminium oder Zinn als Elemente oder in Form ihrer Verbindungen als Katalysator und in Gegenwart eines Adsorbens für schwerlösliche Katalysator-Umwandlungsprodukte in einer Menge von auf 100 Gewichtsteilen Reaktionsansatz 0,1 bis 5, vorzugsweise 0,5 bis 1,5 Gew.-Teile unter Entfernung des gebildeten Wassers, **dadurch gekennzeichnet, dass** man die überschüssige Monocarbonsäure destillativ abtrennt und dem erhaltenen Rohester Wasser bei einer Temperatur unterhalb des Siedepunktes von Wasser bei dem jeweiligen Druck zusetzt und den mit Wasser versetzten Rohester unter Vermeidung von basisch reagierenden Verbindungen nachbehandelt und von den schwerlöslichen Umwandlungsprodukten und dem in der Veresterungsreaktion anwesenden Adsorbens filtriert.

2. Verfahren gemäß Anspruch 1, **dadurch gekennzeichnet, dass** man Wasser in einer Menge von 0,5 bis 5 Gew.-%, vorzugsweise von 1 bis 4 Gew.-%, jeweils bezogen auf den nachzubehandelnden Polyolester, zusetzt.

3. Verfahren gemäß Anspruch 1 oder 2, **dadurch gekennzeichnet, dass** die Nachbehandlung mit Wasser unter Normaldruck bei einer Temperatur von 40 bis unterhalb 100°C, vorzugsweise von 60 bis 90°C erfolgt.

4. Verfahren gemäß Anspruch 1 oder 2, **dadurch gekennzeichnet, dass** die Nachbehandlung mit Wasser oberhalb von Normaldruck und bei mindestens 100°C erfolgt.

5. Verfahren gemäß einem oder mehreren der Ansprüche 1 bis 4, **dadurch gekennzeichnet, dass** man die Nachbehandlung mit Wasser über einen Zeitraum von 10 Minuten bis 4 Stunden, vorzugsweise von 30 Minuten bis 2 Stunden durchführt.

6. Verfahren gemäß einem oder mehreren der Ansprüche 1 bis 5, **dadurch gekennzeichnet, dass** man das Wasser für die Nachbehandlung verwendet, das durch Kondensation von zur Wärme- oder Vakuumerzeugung eingesetztem Wasserdampf zurückgewonnen wird.

7. Verfahren gemäß einem oder mehreren der Ansprüche 1 bis 6, **dadurch gekennzeichnet, dass** sich an die Nachbehandlung mit Wasser eine weitere Behandlung mit Wasserstoffperoxid und unmittelbar folgend eine Wasserdampfbehandlung anschließt.

8. Verfahren gemäß einem oder mehreren der Ansprüche 1 bis 7 zur Nachbehandlung von Triethylenglykol-di-2-ethylhexanoat, Tetraethylenglykol-di-n-heptanoat, Triethylenglykol-di-2-ethylbutyrat, Triethylenglykol-di-n-heptanoat oder Tetraethylenglykol-di-2-ethylhexanoat.

9. Verfahren gemäß einem oder mehreren der Ansprüche 1 bis 8 **dadurch gekennzeichnet, dass** das Adsorbens ausgewählt ist aus der Gruppe der oberflächenreichen Polykieselsäuren wie Silicagele, Kieselgel, Kieselguhr, oberflächenreiche Aluminiumoxide und Aluminiumoxidhydrate, mineralische Materialien, wie Tone oder Carbonate oder Aktivkohle.

## Claims

1. Batchwise process for aftertreatment of polyol esters prepared by reacting polyols of the general formula (II)
H-(-O-[-CR¹R²-]ₘ-)ₒ-OH (II)
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is an integer from 1 to 10, preferably 1 to 8 and especially 1, 2, 3 or 4, o is an integer from 2 to 15, preferably 2 to 8 and especially 2, 3, 4 or 5, with excess linear or branched aliphatic monocarboxylic acids having 3 to 20 carbon atoms that have a lower boiling point than the polyols used, in the presence of a Lewis acid selected from the group of titanium, zirconium, hafnium, iron, zinc, boron, aluminum and tin as elements or in the form of compounds thereof as catalyst and in the presence of an adsorbent for sparingly soluble catalyst conversion products in an amount of 0.1 to 5, preferably 0.5 to 1.5, parts by weight per 100 parts by weight of a reaction mixture, while removing the water formed, **characterized in that** the excess monocarboxylic acid is removed by distillation and water is added to the crude ester obtained, at a temperature below the boiling point of water at the particular pressure and the crude ester with added water is aftertreated with avoidance of basic compounds, and the sparingly soluble conversion products and the adsorbent present in the esterification reaction are filtered off.

2. Process according to Claim 1, **characterized in that** water is added in an amount of 0.5% to 5% by weight, preferably of 1% to 4% by weight, based in each case on the polyol ester to be aftertreated.

3. Process according to Claim 1 or 2, **characterized in that** the aftertreatment with water is effected under standard pressure at a temperature of 40 to below 100°C, preferably of 60 to 90°C.

4. Process according to Claim 1 or 2, **characterized in that** the aftertreatment with water is effected above standard pressure and at at least 100°C.

5. Process according to one or more of Claims 1 to 4, **characterized in that** the aftertreatment with water is conducted over a period of 10 minutes to 4 hours, preferably of 30 minutes to 2 hours.

6. Process according to one or more of Claims 1 to 5, **characterized in that** the water which is recovered by condensation of steam used to generate heat or reduced pressure is used for the aftertreatment.

7. Process according to one or more of Claims 1 to 6, **characterized in that** the aftertreatment with water is followed by a further treatment with hydrogen peroxide, immediately followed by a steam treatment.

8. Process according to one or more of Claims 1 to 7 for aftertreatment of triethylene glycol di-2-ethylhexanoate, tetraethylene glycol di-n-heptanoate, triethylene glycol di-2-ethylbutyrate, triethylene glycol di-n-heptanoate or tetraethylene glycol di-2-ethylhexanoate.

9. Process according to one or more of Claims 1 to 8, **characterized in that** the adsorbent is selected from the group of the high-surface area polysilicic acids such as silica gels, kieselguhr, high-surface area aluminum oxides and aluminum oxide hydrates, mineral materials such as clays or carbonates or activated carbon.

## Revendications

1. Procédé discontinu pour le post-traitement d'esters de polyols, fabriqués par mise en réaction de polyols de la formule générale (II) :
H-(-O-[-CR¹R²-]ₘ-)ₒ-OH (II)
dans laquelle R¹ et R² signifient indépendamment l'un de l'autre hydrogène, un radical alkyle de 1 à 5 atomes de carbone, de préférence méthyle, éthyle ou propyle, ou un radical hydroxyalkyle de 1 à 5 atomes de carbone, de préférence le radical hydroxyméthyle, m signifie un nombre entier de 1 à 10, de préférence de 1 à 8, et notamment 1, 2, 3 ou 4, o signifie un nombre entier de 2 à 15, de préférence de 2 à 8, et notamment 2, 3, 4 ou 5, avec des acides monocarboxyliques aliphatiques linéaires ou ramifiés de 3 à 20 atomes de carbone en excès, qui présentent un point d'ébullition plus faible que les polyols utilisés, en présence d'un acide de Lewis choisi dans le groupe constitué par le titane, le zirconium, l'hafnium, le fer, le zinc, le bore, l'aluminium ou l'étain en tant qu'éléments ou sous la forme de leurs composés en tant que catalyseur, et en présence d'un adsorbant pour les produits de transformation du catalyseur difficilement solubles en une quantité, pour de 100 parties en poids de préparation de réaction, de 0,1 à 5, de préférence de 0,5 à 1,5 partie en poids, avec élimination de l'eau formée, **caractérisé en ce que** l'acide monocarboxylique en excès est séparé par distillation, et de l'eau est ajoutée à l'ester brut obtenu à une température inférieure au point d'ébullition de l'eau à la pression respective, et l'ester brut mélangé avec l'eau est post-traité en évitant les composés réagissant par voie basique, et filtré pour éliminer les produits de transformation difficilement solubles et l'adsorbant présent dans la réaction d'estérification.

2. Procédé selon la revendication 1, **caractérisé en ce que** de l'eau est ajoutée en une quantité de 0,5 à 5 % en poids, de préférence de 1 à 4 % en poids, à chaque fois par rapport à l'ester de polyol à post-traiter.

3. Procédé selon la revendication 1 ou 2, **caractérisé en ce que** le post-traitement avec de l'eau a lieu à pression normale, à une température de 40 à moins de 100 °C, de préférence de 60 à 90 °C.

4. Procédé selon la revendication 1 ou 2, **caractérisé en ce que** le post-traitement avec de l'eau a lieu au-dessus de la pression normale et à au moins 100 °C.

5. Procédé selon une ou plusieurs des revendications 1 à 4, **caractérisé en ce que** le post-traitement avec de l'eau est réalisé pendant une durée de 10 minutes à 4 heures, de préférence de 30 minutes à 2 heures.

6. Procédé selon une ou plusieurs des revendications 1 à 5, **caractérisé en ce que** l'eau qui a été récupérée par condensation de la vapeur d'eau utilisée pour la génération de chaleur ou de vide est utilisée pour le post-traitement.

7. Procédé selon une ou plusieurs des revendications 1 à 6, **caractérisé en ce qu'**un traitement supplémentaire avec du peroxyde d'hydrogène et, directement après, un traitement à la vapeur d'eau, suivent le post-traitement avec de l'eau.

8. Procédé selon une ou plusieurs des revendications 1 à 7, pour le post-traitement de di-2-éthylhexanoate de triéthylène glycol, de di-n-heptanoate de tétraéthylène glycol, de di-2-éthylbutyrate de triéthylène glycol, de di-n-heptanoate de triéthylène glycol ou de di-2-éthylhexanoate de tétraéthylène glycol.

9. Procédé selon une ou plusieurs des revendications 1 à 8, **caractérisé en ce que** l'adsorbant est choisi dans le groupe constitué par les acides polysiliciques de grande surface, tels que les gels de silices, la terre de diatomée, les oxydes d'aluminium et les oxyhydrates d'aluminium de grande surface, les matériaux minéraux, tels que les argiles ou les carbonates ou le charbon actif.
